Europäisches Patentamt

European Patent Office

Office européen des brevets

(11) Publication number: **0 266 035**
**A1**

(12) # EUROPEAN PATENT APPLICATION

(21) Application number: **87307584.0**

(51) Int. Cl.⁴: **A61F 2/06**

(22) Date of filing: **27.08.87**

(30) Priority: **02.09.86 GB 8621150**

(43) Date of publication of application:
**04.05.88 Bulletin 88/18**

(84) Designated Contracting States:
**BE CH DE FR GB IT LI NL SE**

(71) Applicant: **ETHICON INC.**
**U.S. Route 22**
**Somerville New Jersey 08876(US)**

Applicant: **THE UNIVERSITY OF LIVERPOOL**
**First Floor Duncan Building Royal Liverpool**
**Hospital P.O. Box 147**
**Liverpool L69 3BX(GB)**

(72) Inventor: **Berry, John Phillip**
**5 Victoria Drive West Kirby**
**Wirral Merseyside L48 0QU(US)**
Inventor: **Annis, David**
**Little Hey Dibbinsdale Road**
**Bromborough Merseyside L63 0QU(US)**

(74) Representative: **Howick, Nicholas Keith et al**
**CARPMAELS & RANSFORD 43 Bloomsbury**
**Square**
**London WC1A 2RA(GB)**

(54) **Improvements in synthetic vascular grafts.**

(57) A synthetic vascular graft has an inner porous layer, an intermediate substantially non-porous layer (20) and a porous outer layer, in the preferred embodiment the graft being formed by continuous electrostatic spinning.

FIG. 6

EP 0 266 035 A1

## IMPROVEMENTS IN SYNTHETIC VASCULAR GRAFTS

The invention relates to synthetic vascular grafts, and more particularly but not exclusively to tubular synthetic vascular grafts.

Various synthetic vascular grafts have been proposed in the past, from woven and knitted fibrous structures to electrostatically spun fibrous structures. It has been proposed in the past to vary the structure of the fibrous grafts across the thickness of the structure but there has been a continuous search for improved structures having desirable tissue and blood compatability characteristics, as well as desirable physical characteristics, such as resistance to kinking and improved strength characteristics.

According to the invention there is provided a synthetic vascular graft having a first porous layer extending from one surface, a second porous layer extending from a second surface and a substantially non-porous layer lying between said first and second layers.

The graft may be tubular, with the porus layers on the inside and outside of the graft.

The graft is preferably of electrostatically spun polymeric fibres, and preferably formed continuously.

The polymer may be the same throughout the structure, or may be varied during the production process. The substantially non-porous layer may comprise a multiplicity of electrostatically spun fibres collected in conditions where the fibres are not dry of solvent causing melding of those fibres due to the presence of solvent.

The first porous layer may be of different structure from the second porous layer. The first porous layer may comprise at least a portion having a mixture of mircofibres of a first diameter substantially randomly oriented and microfibres of a second diameter larger than the first and arranged substantially circumferentially with respect to the longitudinally axis of the tube, there preferably being circumferentially oriented voids in the first porous layer.

The substantially non-porous layer is preferably nearer the outer surface than the inner surface of the graft.

The inner layer may comprise a plurality of sub-layers, the innermost of which may comprise a microstructure of microfibres generally of said first diameter and generally randomly oriented.

The outermost area may comprise a microstructure of microfibres randomly oriented.

According to the invention there is further provided a method of electrostatically spinning a vascular graft using an electrostatically charged, spinning mandrel and electrostatically charged means in the region of the mandrel to produce an electrostatic field, which method comprises the steps of selecting a desired initial potential for the mandrel, selecting a desired initial potential for the electrostatically charged means and introducing fibreizable material into the electrostatic field produced by the mandrel and the electrostatically charged means to produce a first layer of fibrous structure on the mandrel, altering the spinning conditions such that the fibreizable material arrives at the mandrel with sufficient solvent to cause a substantially non-porous, non-fibrous layer to form on said first layer of fibrous structure, and altering the spinning conditions again to produce a second fibrous layer outside said substantially non-porous, non-fibrous layer.

The spinning conditions are preferably varied by altering the electrostatic field, but may be varied by altering flow rate of fibreizable material into the electrostatic field.

The method may include a further step of altering the spinning conditions during production of the first fibrous layer such that two sub-layers of differing fibrous structures are formed in said first layer.

Where a vascular graft is to be non-tubular, the method may comprise the steps of forming a tubular graft, removing the graft from the mandrel, and cutting the desired dimension of graft from the tube.

By way of example, one embodiment of a synthetic vascular graft and a method of making the graft according to the invention will now be described with reference to the accompanying drawings, in which:-

Figure 1 is a side view of apparatus for spinning a tubular fibrous structure shown in one mode of operation;

Figure 2 is a side view illustrating the apparatus of Figure 1 in a second mode of operation;

Figure 3 is a side view of the apparatus of Figure 1 in a third mode of operation;

Figure 4 is a scanning electron microscope photograph of the microstructure of the surface of a tubular fibrous structure including fibres of different diameters;

Figure 5 is a scanning electron microscope photograph of a section through the microstructure of a tubular fibrous structure having fibres of different diameters;

Figure 6 is a scanning electron microscope photograph of a section through the microstructure of a tubular fibrous structure including a substantially non-porous layer; and

Figure 7 is a scanning electron microscope photograph of the surface of a substantially non-porous layer of a tubular fibrous structure.

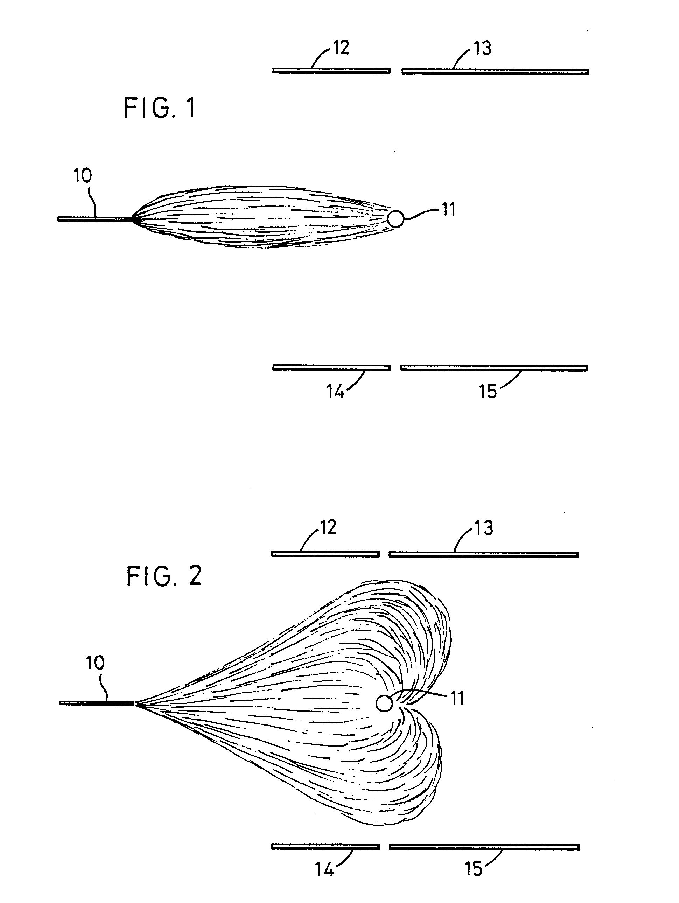

Figures 1 and 2 illustrate the apparatus diagrammatically. The general electrostatic spinning process has been described in several Patent specifications already published, for example earlier published British Application Nos. 2121286A and 2120946A.

The apparatus consists of an array of capillary needles 10 mounted on a carrier (not shown) with means for moving the carrier in reciprocating fashion parallel to a mandrel 11. The means for moving the needle carrier is conveniently a motor.

The mandrel 11 in the embodiment illustrated is of 4mm diameter although it will be appreciated that other diameters may be used depending on the type of vascular graft required. There are means for rotating the mandrel of a variety of different speeds but typically the speed will be of several thousand revolutions per minute.

The capillary needles 10 are supplied with polymeric material such as polyurethane or another suitable polymer in solution (although it is possible to use a suspension) and material emanating from the needles is attracted towards the mandrel 11 by electrostatically charging the mandrel 11 to a potential of several kilovolts with respect to the needles 10. The process results in the production of fibres with collect on the mandrel 11 in a manner which has been described in specifications already published.

It has already been proposed to have electrostatically charges grids on either side of the mandrel 11 with respect to the needles 10 and in the apparatus of Figures 1 and 2, pairs of plates 12, 13 and 14, 15 are provided. The plates 12, 13, 14 and 15 are conveniently of metallic sheeting although barred grids may be used if desired.

The Figure 1 mode of operation in which all four plates 12, 13, 14 and 15 are electrostatically charged to a voltage less than the voltage to which the mandrel 11 is charged is much the same as known electrostatic spinning processes and results in an array of fibres leading from the needles 10 to the mandrel 11. Typical voltages for the particular spacing of the plates and size of the mandrel illustrated in Figure 1 are 12kV on the mandrel and 6kV on the plates to produce a substantially uniform microstructure with fibres of a diameter substantially in the range 0.5μm to 2μm. Thus the Figure 1 mode of operation is substantially that described in previous published specifications.

By changing the mode of operation to that shown in Figure 2, the fibrous structure formed on the mandrel 11 changes substantially. To produce the shape of fibre array emanating from the needles 10 shown in Figure 2, the forward plates 12 and 14 of the two pairs of plates are no longer electrostatically charged but are at the same potential as the needles 10. The rearward plates 13 and 15 have parallel leading edges which lie in a plane passing through or at least adjacent the mandrel 11 and are charged to a higher voltage that was the case in Figure 1. The mandrel voltage is reduced. This causes fibres to be attracted more readily towards the rearward plates 13 and 15 and thence to a mandrel 11. Some fibres will be attracted directly to the mandrel 11. Fine adjustment of the voltages is required to achieve the Figure 2 mode of operation and typical voltages are 9.2kV on the plates 13 and 15 and 7kV on the mandrel 11. It will be appreciated, however, that the voltage relationship between the mandrel 11 and the plates 13 and 15 will differ depending on the plate size and spacing, the mandrel diameter, the spacing of the needles 10 from the mandrel 11 and the plates 12, 13, 14 and 15 and the flow rate of fibreizable material.

The fibrous structure produced in the mode of operation described with reference to and shown in Figure 2 differs significantly from the fibrous structure produced by the mode of operation described with reference to and shown in Figure 1. In the Figure 1 mode of operation, the fibres are generally in the range 0.5μm to 2μm, and mostly of approximately 1μm diameter, and are generally randomly oriented. In the Figure 2 mode of operation, however, while some fibres of diameter of approximately 1μm (in the range 0.5μm to 2μm) are still formed on the mandrel 11 with random orientation, fibres of a larger diameter are also produced, probably from the fibres whose path length to the mandrel 11 is lengthened because of the different electrostatic field. Fibres of a diameter up to 20μm may be produced and, in general, the larger the fibre diameter, the more likely is that fibre to be arranged circumferentially with respect to the longitudinal axis of the mandrel 11. The larger diameter fibres are, in effect, more likely to be wound around the mandrel 11 leading to circumferential orientation.

In general, the Figure 2 mode of operation will be achieved at a low rate significantly higher than the Figure 1 mode of operation.

If, however, the flow rate of the Figure 2 mode of operation is maintained by electrostatic conditions are altered and the mandrel speed is reduced such that the electrostatic conditions are similar to those of the Figure 1 mode of operation but with a mandrel speed reduced to, for example 1000 rpm as opposed to, for example, 5000 rpm for the Figures 1 and 2 modes of operation, fibreizable material is collected on the mandrel 11 in a further different way. Because of the electrostatic field in existance, material is attracted

3

with a short path length and essentially directly to the mandrel 11. However, high flow rates mean that there is insufficient time for solvent containing the polymeric material to evaporate completely and fibres arriving at the mandrel 11 are still substantially wet, resulting in fibres arriving on the mandrel 11 forming a meld to provide a continuous and substantially non-porous layer around the mandrel 11.

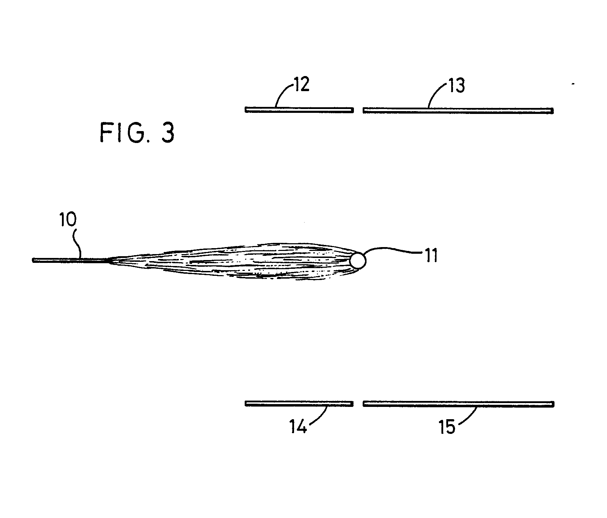

The Figure 3 mode of operation may be used in providing a substantially non-porous layer in a tubular fibrous graft. An example of the method of producing such a graft with a non-porous layer in is as follows:-

1. Initial formation of a fibrous structure is carried out in the Figure 2 mode of operation with the mandrel voltage at 6.9kv, the voltage of grids 13 and 15 at 9kV and mandrel speed at 5000 rpm.

2. When half the total volume of fibreizable material has been deposited, the mandrel voltage is increased from 6.9kV to 11kV, the grid voltage is reduced from 9kV to 6kV and the mandrel speed is reduced to 1000 rpm, producing the Figure 3 mode of operation. These conditions are maintained for 20 minutes, the flow rate being unchanged.

3. After 20 minutes, conditions are returned to those set out in paragraph 1 above.

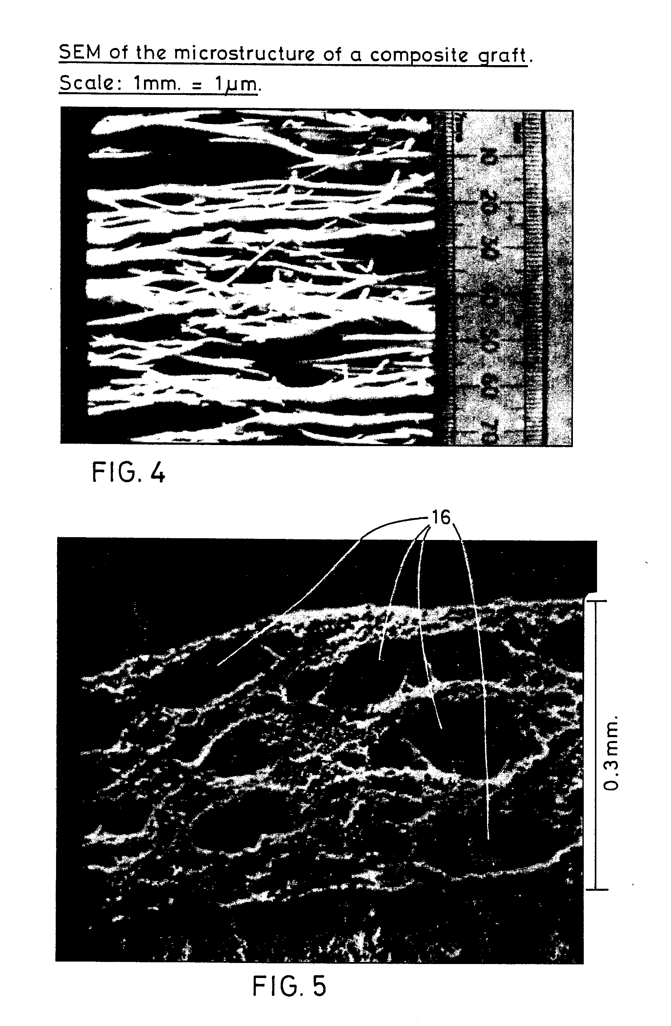

Figures 4, 5, 6 and 7 illustrate the sorts of structure appearing in a tubular vascular graft made by the method just described. Figure 4 illustrates the type of microstructure produced in the Figure 2 mode of operation, it being apparent that fibres of a diameter of approximately 1μm extend randomly in direction over the structure, whereas fibres of larger diameter extend generally circumferentially.

It has been found that the microstructure produced in the Figure 2 mode of operation includes elongate voids in the structure which generally extend circumferentially with respect to the longitudinal axis of the mandrel 11 and the tubular fibrous structure formed on it. Figure 5 illustrates the presence of the voids 16, Figure 5 being a scanning electron microscope photograph of a section through the microstructure. The presence of the voids 16 in the microstructure assists in resisting kinking of the structure upon bending.

Thus the inner layer of a graft produced according to the method example set out above would have a structure as shown in Figures 3 and 4.

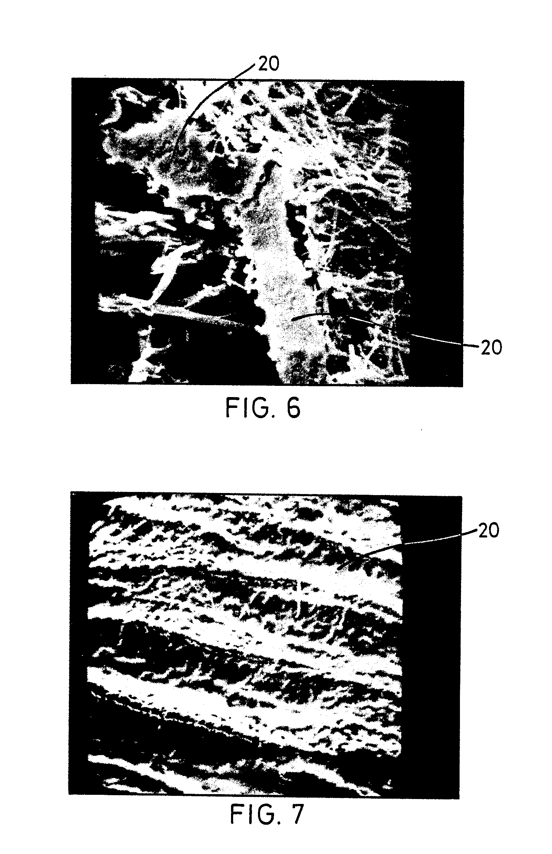

Figure 6 is a cross-sectional view of the non-porous layer indicated by reference numeral 20 in Figure 6. In this particular case, a graft was held in liquid nitrogen for 1 minute and then broken open. A specimen was observed using a scanning electron microscope. The non-porous melding layer can be seen as the smooth, broad band 20 passing from top to bottom. The layer 20 is v-shaped, following a contour on the graft and is approximately 20μm wide. Spun fibres can be seen on either side of the layer 20, indicating that the fibrous structure has not been destroyed in formation of the non-porous layer 20 during arrival of the wet fibres.

Figure 7 shows the surface of the non-porous layer 20, the particular graft illustrated in Figure 7 having been made according to the method set out above but with the spinning process halted immediately after application of the non-porous layer 20. A portion was cut from the graft and this was viewed en face using the scanning electron microscope. As can be seen in Figure 7, the surface of the non-porous layer is very smooth and no fibres are visible, indicating that a melded layer was formed. The nature of the fibrous structure beneath the layer 20 is of the Figure 2 mode with larger circumferential fibres. This is clearly illustrated in Figure 7 with the layer 20 following the contours. Some holes are visible at the peak of each contour which accounts for the final graft being slightly porous although the graft is substantially non-porous as a result of the layer 20.

It will be appreciated that the substantially non-porous layer could be anywhere within the fibrous structure but it is preferred to have the substantially non-porous layer towards the outside of the graft in order to assist physical properties. Part of the attraction of the Figure 2 mode of operation structure including circumferentially oriented voids and larger diameter fibres is the good resistance of such a graft to kinking explained in straightforward terms by the fact that the voids and circumferential large fibres allow without overall distortion of the structure axial compression and elongation. By putting the substantially non-porous layer towards the outside of such a graft, the substantiall non-porous layer follows the contours of the structure including the larger diameter fibres and the undulations thereby produced permit flexure of the tubular structure because the substantially non-porous layer is circumferentially corrugated, elongation of the substantially non-porous layer causing the corrugations to be pulled out and axial compression of the structure causing the corrugations to be accentuated.

It will be appreciated, however, that a substantially non-porous layer could be of assistance in structures of the Figure 1 mode of operation and such a graft could readily be produced by changing the conditions to those set out above for the Figure 3 mode of operation during production of an otherwise Figure 1 mode of production graft.

The presence of a substantially non-porous layer in a graft has a general effect on physical properties as illustrated in the table below in which a graft solely of the Figure 2 mode of production is compared to a graft of the Figure 2 mode of production but including a substantially non-porous layer made by the Figure 3 mode of production although both have an inner fibrous sub-layer of approximately 1000μm of Figure 1 mode of production. For the graft including the Figure 3 mode layer, the bulk of the inner fibrous layer was formed on a mandrel of 6.3mm diameter rotating at 5000 rpm, the mandrel voltage being 66.9kV and the grid voltage being 9.2kV. The substantially non-porous layer was operated with the same flow rate but with the mandrel rotating at 1000 rpm, the mandrel voltage being 12kV and the grid voltage being 6kV. The outer fibrous layer was produced with the mandrel speed of rotation being 1000 rpm, the mandrel voltage being 6.9kV and the grid voltage being 9.2kV. The physical property comparison is as follows:-

## PHYSICAL PROPERTY COMPARISON

|  | Fig. 2 mode graft. | Fig. 2 mode graft with Fig. 3 mode layer. |
|---|---|---|
| Density (gms/cc) | 0.2171 | 0.2764 |
| Wt. per cm (gm/cm) | 0.0518 | 0.0538 |
| Internal diameter (mm) | 5.848 | 5.8133 |
| Outer diameter (mm) | 8.036 | 7.6533 |
| Straight kink diameter (mm) | | |
| at    0mm Hg | 16mm | 46mm |
| 15mm Hg | 15mm | 36mm |
| 100mm Hg | 15mm | 32mm |
| 150mm Hg | 15mm | 31mm |
| Porosity (ml/cm$^2$-min) | 54.26 | 28.02 |
| Suture pullout (gms) | 476.5 | 587.9 |
| Circumferential ultimate stress (kg/cm$^2$) | 34.565 | 49.523 |
| Circumferential modulus (kg/cm$^2$) | 11.035 | 12.457 |
| Longitudinal ultimate stress (kg/cm$^2$) | 9.632 | 21.181 |
| Longitudinal modulus (kg/cm$^2$) | 2.709 | 4.099 |

It will be appreciated that many different combinations of mandrel size, mandrel to needle spacing, grid spacing and potentials could be used to create a substantially non-porous layer and that the foregoing example is merely illustrative.

The advantages of the graft produced are that the porosity of the Figure 2 mode graft is substantially reduced by the presence of the substantially non-porous layer, passage of blood plasma through the graft being substantially prevented on first contact with blood. The strength properties are enhanced and flexibility is not impaired significantly. Excessive ingrowth of living tissue into the graft from outside is prevented due to the substantially impervious layer. The result is a graft which has attractive properties as a synthetic vascular graft.

**Claims**

1. A synthetic vascular graft having a first porous layer extended from one surface, a second porous layer extending from a second surface and a substantially non-porous layer lying between said first and second layers.

2. A graft as claimed in Claim 1 being tubular with the porous layers on the inside and the outside of the graft.

3. A graft as claimed in Claim 1 or Claim 2 formed of electrostatically spun polymeric fibres.

4. A graft as claimed in Claim 3 when the graft is formed by a continuous electrostatic spinning process.

5. A graft as claimed in any preceding Claim wherein the polymer is the same throughout the structure.

6. A graft as claimed in any one of Claims 1 to 4 wherein the polymer varies in composition in the structure.

7. A graft as claimed in any preceding Claim wherein the substantially non-porous layer comprises a multiplicity of electrostatically spun fibres collected in conditions where the fibres are not dry of solvent, causing melding of those fibres due to the presence of solvent.

8. A graft as claimed in any preceding Claim wherein the first porous layer is of different structure from the second porous layer.

9. A graft as claimed in Claim 8 wherein the first porous layer comprises at least a portion having a mixture of microfibres of a first diameter substantially randomly oriented and microfibres of a second diameter larger than the first and arranged substantially circumferentially with respect to the longitudinal axis of the tube.

10. A graft as claimed in Claim 9 wherein said portion including microfibres of said first and second diameters further includes circumferentialy oriented voids.

11. A graft as claimed in Claim 9 or Claim 10 wherein the inner layer comprises a plurality of sub-layers, the innermost of which comprises a microstructure of microfibres generally of said first diameters and generally randomly oriented.

12. A graft as claimed in any preceding Claim wherein the outermost area comprises a microstructure of microfibres randomly oriented.

13. A graft as claimed in any preceding Claim wherein the substantially non-porous layer is nearer the outer surface than the inner surface of the graft.

14. A method of electrostatically spinning a vascular graft using an electrostatically charged, spinning mandrel and electrostatically charged means in the region of the mandrel to produce an electrostatic field, which method comprises the steps of selecting a desired initial potential for the mandrel, selecting a desired initial potential for the electrostatically charged means and introducing fibreizable material into the electrostatic field produced by the mandrel and the electrostatically charges means to produce a first layer of fibrous structure on the mandrel, altering the spinning conditions such that the fibreizable material arrives at the mandrel with sufficient solvent to cause a substantially non-porous, non-firbrous layer to form on said first layer of fibrous structure, and altering the spinning conditions again to produce a second fibrous layer outside said substantially non-porous, non-fibrous layer.

15. A method as claimed in 14 wherein the spinning conditions are varied by altering the electrostatic field.

16. A method as claimed in Claim 14 wherein the spinning conditions are varied by altering flow rate of fibreizable material into the electrostatic field.

17. A method as claimed in any one of Claims 14 to 16 including a further step of altering the spinning conditions during production of the first fibrous layer such that two sub-layers of differing fibrous structure are formed in said first layer.

18. A method as claimed in any one of Claims 14 to 17 for producing a non-tubular vascular graft, which method comprises the further step of removing the graft from the mandrel and cutting the desired dimension of graft from the tube.

FIG. 1

FIG. 2

FIG. 3

SEM of the microstructure of a composite graft.
Scale: 1mm. = 1μm.

FIG. 4

FIG. 5

FIG. 6

FIG. 7

## DOCUMENTS CONSIDERED TO BE RELEVANT

| Category | Citation of document with indication, where appropriate, of relevant passages | Relevant to claim | CLASSIFICATION OF THE APPLICATION (Int. Cl.3) |
|---|---|---|---|
| A | GB-A-2 092 894 (THORATEC LAB. CORP.) <br> * figure 1; claims 4-6 * | 1 | A 61 F  2/06 |
| A | DE-A-2 806 030 (INSTITUTE FÜR TEXTIL- UND FASERFORSCHUNG STUTTGART) <br> * figure 2; claims 1,15,23,30 * | 1 | |
| A | GB-A-2 142 870 (ETHICON INC. et al.) <br> * claims 10,19; figures * | 1,3,14 | |
| A | EP-A-0 009 941 (ICI LTD.) <br> * claims 1,15,22; figures * | 1,3,14 | |
| D,A | GB-A-2 121 286 (ETHICON INC. et al.) <br> * claims 1,12; figure 1 * | 1,3,14 | |

TECHNICAL FIELDS
SEARCHED (Int. Cl.3)

A 61 F  2/00

The present search report has been drawn up for all claims

| Place of search | Date of completion of the search | Examiner |
|---|---|---|
| BERLIN | 14-12-1987 | KANAL P K |